# EUROPEAN PATENT APPLICATION

(11) **EP 2 108 645 A1**
(43) Date of publication of application: **14.10.2009**
(21) Application number: 08154159.1
(22) Date of filing: 07.04.2008
(51) Int. Cl.: C07D 311/86, A61K 31/352, A61P 35/00

(54) **Use of xanthone derivatives as a medicament for cancer**

(71) Applicant: INSERM (Institut National de la Santé et de la Recherche Medicale), 75013 Paris (FR)
(72) Inventor: The designation of the inventor has not yet been filed
(74) Representative: Jacobson, Claude

(57) **Abstract**

The invention relates to xanthone derivatives of formulae (IA) or (IB) and their use for manufacturing a medicament for cancer, and in particular for chemotherapeutic resistant cancer. More particularly, the invention relates to compounds and compositions comprising such xanthone derivatives for the prevention and/or treatment for chronic leukemia and, for example, for chronic lymphocytic leukemia (CLL) or B-lymphoma. 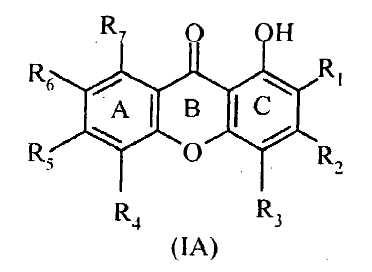 or

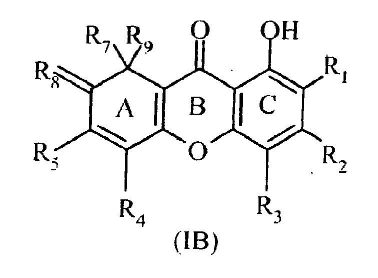

## Description

The instant invention relates to xanthone derivatives and their use for manufacturing a medicament for cancer, and in particular for chemotherapeutic resistant cancer. More particularly, the invention relates to compounds and compositions comprising such xanthone derivatives for the prevention and/or treatment for chronic leukemia and, for example, for chronic lymphocytic leukemia (CLL) or B-lymphoma.

It is well acknowledged that a defect in apoptosis (or programmed cell death) is a key element in tumor progression as it may result in a decrease or even more in an absence of efficiency of anti-tumoral therapy relying upon the induction of apoptosis into tumor cells.

Leukemias are cancers of the white blood cells involving bone marrow, circulating white blood cells and organs such as spleen and lymph nodes. Malignant transformations usually occurs at the pluripotent stem cells level, although it sometimes involves a committed stem cells with more limited capacity for differentiation. Abnormal proliferation, clonal expansion and diminished apoptosis (programmed cell death) lead to replacement of normal blood elements with malignant cells.

Leukemias were originally termed acute or chronic, based on life expectancy, but now are classified according to cellular maturity.

Chronic leukemias are described as lymphocytic (CLL) or myelocytic (CML).

B cell chronic lymphocytic leukemia (B-CLL) is the most frequent leukemia in Western countries and, despite recent progress, still remains an incurable disease (Banerji et al, Curr. Opin. Oncol., 2000; 12: 22-29). At variance with other leukemias, B-CLL cells do not express a characteristic translocation, although about 80% of the patients exhibit various cytogenetic alterations. A small pool of highly proliferating cells has been identified in the bone marrow and lymph nodes and is believed to feed the blood compartment (Messmer et al, J. Clin. Invest., 2005; 115: 755-764). The latter consists in anergic and non-dividing small B lymphocytes, 95-98% of them being arrested in the G₀/G₁ phase of the cell cycle. Although these cells are highly resistant to apoptosis *in vivo*, they become sensitive when cultured *ex vivo* and die rather rapidly, unless they are incubated in the presence of stromal cells that can rescue them from programmed cell death. This suggests that their micro-environment protect them from apoptosis induction *in vivo* and therefore B-CLL is a disease of both proliferation and accumulation.

In the so-called "indolent patients" with stable lymphocyte counts, there exists a homeostatic balance between proliferation and accumulation, whereas an imbalance exists in "severe patients" with increasing lymphocyte counts (Chiorazzi, Best Pract Res Clin Hematol 2007; 20: 399-413).

An efficient therapeutic approach of B-CLL should thus combine the development of new agents able to revert the resistance to apoptosis of the leukemic cells, but also to block the replication of the small pool of highly dividing cells present in the proliferation centers of the bone marrow and lymph nodes.

The search for new agents able to counteract this resistance to apoptosis is significant to consider new therapeutic approaches.

Plant of the genus *Allanblackia* have been subjected to phytochemical investigations as they display a great structural variability.

*Allanblackia sp*. belongs to the family of Guttiferae and are used in traditional medicine for the treatment of respiratory infections, diarrhoea and toothache (Raponda-Walker et al, Les plantes utiles du GABON, Paul LECHEVALIER, Paris VI, 1961).

Phytochemical studies of plants belonging to the genus *Allanblackia* have revealed the presence of xanthones, benzophenones, biflavonoids, phytosterols and saponins (Locksley et al, J. Chem. Soc. C 1971; 1332-1340; Blunt et al., J. Nat. Prod. 1999; 62: 130-132; Nkengfack et al., Phytochemistry 2002; 60: 381-384). Some of these compounds exhibit a wide range of biological and pharmacological activities such as cytototoxic, anti-inflammatory, antimicrobial and antifungal (Peres et al, Phytochemistry. 2000; 55: 683-710), as well as HIV inhibitory activity (Blunt et al., J. Nat. Prod. 1999; 62: 130-132).

Macluraxanthone was found to display cytotoxic activity on human A549 lung cancer cells with an IC50=2.88 µM and SK-OV3 ovarian cancer cells with an IC50=4.24 µM (Lee et al, J Nat Prod. 2005; 68: 456-458).

Norcowanin was reported to present antiplasmodial activity, as well as weak cytotoxicity on the human A375 cell line (Azebaze et al, Chem Pharm Bull. 2006; 54:111-113).

α-mangostin is an histamine H1 receptor antagonist (Chairungsrilerd et al., Eur J Pharmacol. 1996; 314: 351-356) that displays antiplasmodial activity (Mahabusarakam et al, Planta Med. 2006; 72: 912-916; Azebaze et al., Ann Trop Med Parasitol. 2007; 101:23-30), induces apoptosis in human promyelocytic leukemia HL-60 cell line (Matsumoto et al., J Nat Prod. 2003; 66: 1124-1127) and induces cell-cycle arrest and apoptosis in human colon cancer DLD-1 cells by affecting the expression of cyclins, cdc2 and increasing p27 (Matsumoto, Bioorg Med Chem. 2005; 13: 6064-6069).

Allanxanthone C displays antiplasmodial activity as well as cytotoxicity on the human A375 melanoma cell line (Azebaze et al., Chem Pharm Bull. 2006; 54: 111-113).

There is a need to provide novel agent able to re-sensitize tumor cells to apoptosis process.

There is a need to provide novel agents able to prevent and/or reduce the resistance of tumor cells to chemotherapy or radiotherapy.

There is a need to provide novel agents suitable for treating and/or preventing cancer and at the same time which are not deleterious to normal healthy cells.

In particular, there is a need to sensitize tumor cells from B-CLL or B-lymphoma to chemotherapy or radiotherapy without affecting normal white blood cells.

There is a need to provide novel agents able to induce apoptosis into tumor cells as well as to prevent or reduce abnormal excessive proliferation of said tumor cells.

The instant invention has for object to meet these needs.

The inventors have unexpectedly observed that some xanthone derivatives, in particular some xanthone derivatives extracted from the genus *Allanblackia,* displayed apoptotic and antiproliferative activities on cells from B-cell chronic lymphocytic leukemias.

More particularly, the inventors have surprisingly observed that a series of xanthone derivatives isolated from *Allanblackia monticola* and from related plants such as *Allanblackia floribunda, Allanblackia gabonensis* and *Calophyllum inophyllum* and named thereafter M1, M2, M3, M4, M5 and M6 may have proapoptotic and antiproliferative effects on B-CLL cells.

In particular, it has been observed that those molecules display potent anti-proliferative activity on the growth of two cell lines isolated from patients with chronic B cell malignancies, hairy cell leukemia (HCL) and B-cell chronic lymphocytic leukemia (B-CLL) as well as on tumour cells freshly isolated from B-CLL patients and cultured *ex vivo*. This inhibition of the replication of the leukemic cells was correlated with an impairment of the viability and the induction of apoptosis in both cell lines and in freshly isolated tumour cells, as evidenced by the characteristic internucleosomal cleavage of DNA.

Surprisingly, the inventors have observed that those xanthone derivatives did not significantly impair the viability of normal PBMC and B lymphocytes.

According to one of its objects, the invention relates to a xanthone derivative of general formula (IA) or (IB): 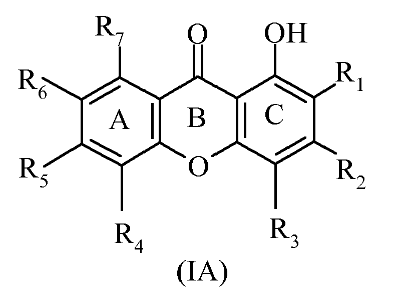 or 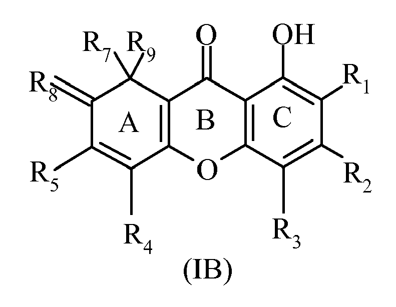 wherein
- R₁, R₃, R₇ and R₉ are, independently of each other, H or a linear, branched or cyclic, saturated or unsaturated, C₁-C₁₂ alkyl group, with the provisio that at least one of R₁, R₃ or R₇ is an alkyl group as above-defined,
- R₂, R₄ and R₅ are, independently of each other, H, -OH, -NH₂ or -SH, or
- R₁ and R₂ form together a 5 to 7-membered ring fused with ring C, said ring being saturated or unsaturated, and optionally comprising at least one heteroatom chosen from O, N or S, and optionally being substituted with one or more linear, branched or cyclic, saturated or unsaturated, C₁-C₆ alkyl groups and R₃, R₄, R₅, R₇ and R₉ being as above-defined,
- R₆ is a group chosen among linear or branched, saturated or unsaturated, C₁-C₄ alcoxy, C₁-C₄ alkyl- or dialkyl-amino, or C₁-C₄ alkyl-imido groups,
- R₈ is chosen among an oxygen, an imine or a thioether,
or a pharmaceutically acceptable salt, an ester, an ether or an isoform thereof, or a mixture thereof,
for use as a medicament for the prevention and/or the treatment of a cancer chosen from chronic leukemias.

One object of the invention is the use of a xanthone derivative of the invention for the manufacture of a medicament intended to prevent and/or treat a cancer, in particular a cancer chosen from chronic leukemias.

The xanthone derivatives are used in a medicament of the invention as active agent.

Within the meaning of the invention, the terms "prevention" or "preventing" are to be understood as suppressing or reducing the risk of occurrence of an event.

Within the meaning of the invention, the terms "treatment" or "treating" with respect to a disease condition are to be understood as curing or alleviating or reducing symptoms of said disease condition.

In one embodiment, the chronic leukemia may be chosen from B-cells Chronic Lymphoid Leukemia (B-CLL) or B-lymphoma.

According to one embodiment, the cancer may be resistant to chemotherapy or radiotherapy.

According to another embodiment, a medicament of the invention may be intended to be administered separately, sequentially or simultaneously with a chemotherapeutic agent or a radiotherapeutic regimen.

According to another embodiment, a medicament of the invention may be intended to sensitize cancer cells to a chemotherapeutic agent or a radiotherapeutic regimen.

According to one of its object, the invention relates to a kit-of-parts comprising (i) a xanthone derivative according to the invention, and (ii) a chemotherapeutic agent, each of (i) and (ii) being laid out in a separate dosage form unit.

According to one embodiment, a kit-of-part of the invention may be configured such that the (i) xanthone derivative of the invention and the (ii) chemotherapeutic agent may be laid out to be administered separately, sequentially or simultaneously.

According to another of its object, the instant invention relates to a method for preventing and/or treating a cancer chosen from chronic leukemias comprising at least the step of administering to an individual in need thereof at least an effective amount of at least one xanthone derivative according to the invention.

Within the meaning of the invention, the terms "effective amount" are to be understood as meaning the required and sufficient amount needed to observe a given effect, such as for example, in the context of the invention, the reduction of number of tumor cells within a context of preventing and/or treating a cancer condition.

According to one of its advantages, the use of xanthone derivatives in accordance with the invention may increase quality of life of cancerous patients.

According to one of its advantages, the use of xanthone derivatives in accordance with the invention may offer alternative treatment with respect to usual treatment of chronic leukemias.

According to one of its advantages, the use of the xanthone derivatives with radiotherapy or chemotherapy in accordance with the invention, allows to decrease the required dose of radiotherapeutic radiation or chemotherapeutic agent.

According to one of its advantages, the use of the xanthone derivatives with radiotherapy or chemotherapy in accordance with the invention allows to reduce or even suppress the side effects of radiotherapy or chemotherapy.

### XANTHONE DERIVATIVES

A xanthone derivative according to the invention may be of the following general formula (IA) or (IB): 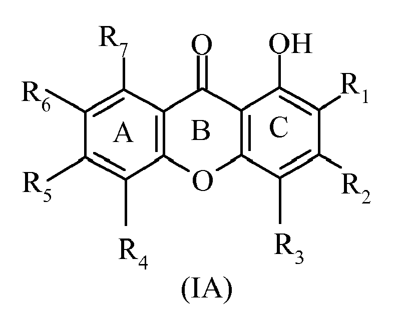 or 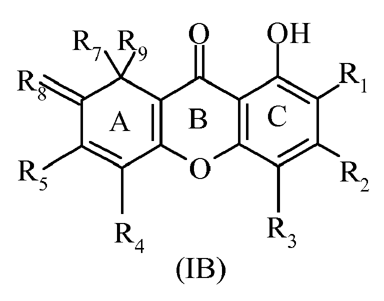 wherein
- R₁, R₃, R₇ and R₉ are, independently of each other, H or a linear, branched or cyclic, saturated or unsaturated, C₁-C₁₂ alkyl group, with the proviso that at least one of R₁, R₃ or R₇ is an alkyl group as above-defined,
- R₂, R₄ and R₅ are, independently of each other, H, -OH, -NH₂ or -SH, or
- R₁ and R₂ form together a 5 to 7-membered ring fused with ring C, said ring being saturated or unsaturated, and optionally comprising at least one heteroatom chosen from O, N or S, and optionally being substituted with one or more linear, branched or cyclic, saturated or unsaturated, C₁-C₆ alkyl groups, and R₃, R₄, R₅, R₇ and R₉ being as above defined,
- R₆ is a group chosen among linear or branched, saturated or unsaturated, C₁-C₄ alcoxy, C₁-C₄ alkyl- or dialkyl-amino, or C₁-C₄ alkyl-imido groups,
- R₈ is chosen among an oxygen, an imine or a thioether.

The invention also relates to pharmaceutically acceptable salts, ester, ether or isoform of the xanthone derivatives in accordance with the invention.

The xanthone derivatives of the invention may be used alone or in mixtures. They may be used as a plant extract or after purification according to any known methods in the art.

Within the meaning of the invention, the term "isoform" is intended to mean tautomers, stereoisomers, polymorphous forms or pharmaceutically acceptable solvates.

The term "tautomer" is intended to mean isomers, the structure of which differ by the position of one atom, typically one hydrogen atom, and one or more multiple bonds and which are able to easily and reversibly transform into each other.

The term "stereoisomer" is intended to mean isomers from a molecule which are identical in constitution but which differ only by one or more different arrangements of their atoms in space.

The terms "pharmaceutically acceptable salts" is intended to mean a compound which may be obtained by reaction of a compound of general formula (IA) or (IB) with a base or an acid.

As illustrative examples of bases that may be suitable for the invention, one may mention sodium hydroxide, sodium methoxide, sodium hydrate, potassium t-butoxide, calcium hydroxide, magnesium hydroxide and the like, and their mixtures in solvent such as THF (tetrahydrofuran), methanol, t-butanol, dioxane, isopropanol, ethanol and the like, and their mixtures.

Organic base such as lysine, arginine, diethanolamine, choline, tromethamine, guanidine, and the likes, may also be used.

As examples of acid suitable for the invention, one may mention chlorhydric acid, bromhydric acid, nitric acid, sulfuric acid, phosphoric acid, p-toluenesulfonic acid, methane sulfonic acid, acetic acid, citric acid, maleic acid, salicylic acid, hydroxynaphtoic acid, ascorbic acid, palmitic acid, succinic acid, benzoic acid, benzene sulfonic acid, tartric acid and the like, and their mixtures, in solvent such as ethylacetate, ether, alcohol solvent, acetone, THF, dioxane, and the like and their mixtures.

The terms "polymorphous form" are intended to mean compounds obtained by crystallization of a compound of general formula (IA) or (IB) in different conditions, as for example the use of different sequences, usually used for crystallization. Crystallization at different temperature implies for example various mode of cooling, for example very fast to very low cooling implying warming or melting steps of compounds followed by fast or gradual cooling.

The presence of polymorphous forms may be identified by NMR spectroscopy, by IR-spectroscopy (infrared), by differential scanning calorimetry (DSC), by X-ray diffraction or other similar techniques known in the art.

As example of esters of xanthone derivatives according to the invention, one may mention succinate, hemisuccinate, malate, tartrate or glycolate of a compound according to the invention.

Within the meaning of the invention, the term "unsaturated" is intended to mean that the group may comprise one or more double or triple bond(s).

When more than one unsaturated bonds are presents, for example at least two double bonds, they may or may not be conjugated.

According to one embodiment, one or more of R₁, R₃, R₇ or R₉ may be a linear, branched or cyclic, saturated or unsaturated, C₂-C₁₂, C₃-C₁₀ or C₅-C₁₀ alkyl group.

According to another embodiment, one or more of R₁, R₃, R₇ or R₉ may be a 3-methyl-but-2-enyl group or a 3,7-dimethyl-oct-2,6-dienyl group.

According to one embodiment, R₁ may be a 3-methyl-but-2-enyl group and R₃ and R₇ may be H.

According to one embodiment in formula (IA), R₁ may be a methyl-but-2-enyl group, and R₇ may be a 3-methyl-but-2-enyl group or a 3,7-dimethyl-oct-2,6-dienyl group and R₃ may be H.According to one embodiment in formula (IB), R₁ R₇ and R₉ may be a 3-methyl-but-2-enyl group and R₃ may be H.

According to another embodiment, the ring formed by R₁ and R₂ may comprise one or more heteroatom, and preferably at least one oxygen.

According to another embodiment, the ring formed by R₁ and R₂ may comprise at least one unsaturation.

According to one embodiment, the ring formed by R₁ and R₂ may comprise at least two or more double bonds, conjugated or not.

In one embodiment, the ring may be an unsaturated hydrocarbon ring comprising at least one heteroatom, in particular an oxygen atom.

According to another embodiment, the ring may be substituted with one or more linear or branched, saturated or unsaturated, C₁-C₃ alkyl groups.

According to another embodiment, the ring may be substituted with at least one or more methyl groups, and more particularly with at least two methyl groups.

According to another embodiment, R₁ and R₂ may form together a 6-membered ring fused to ring C.

According to one embodiment the 6-membered ring may optionally comprise at least one heteroatom, in particular an oxygen, and/or may optionally be substituted with one or more linear or branched, saturated or unsaturated, C₁-C₃ alkyl groups.

In one embodiment, R₃ is different from hydrogen when R₁ et R₂ form together a six-membered unsaturated ring fused to ring C.

According to another embodiment, at least one of R₂, R₄ or R₅ may be OH.

According to one embodiment, at least R₂ may be OH and R₄ and R₅ may be H.

According to another embodiment, R₂ and R₅ may be OH and R₄ may be H.

According to another embodiment, a xanthone derivative according to the invention may comprise at least two hydroxyl groups and in particular at least three hydroxyl groups, and more particularly at least four hydroxyl groups.

According to another embodiment, R₆ may be a C₁-C₂ alkoxy group, and in particular is a methoxy group.

According to another embodiment, R₈ may be an oxygen.

According to another embodiment, a xanthone derivative according to the invention may be chosen from a compound of formula M1/(II), M2/(III), M4/(IV), M5/(V), M6/(VI) and M3/(VII): 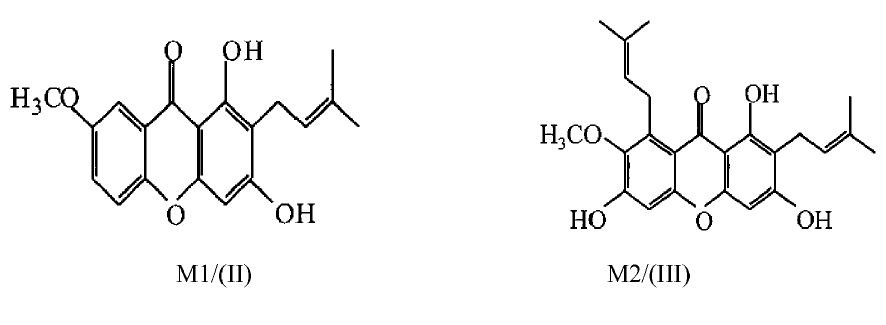 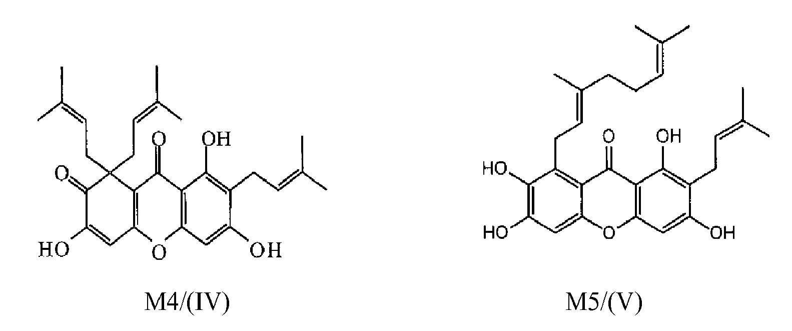 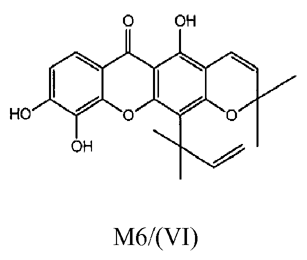 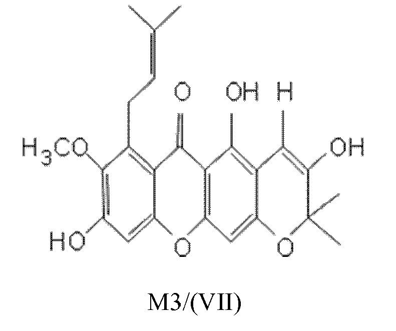 or a pharmaceutically acceptable salt, an ester, an ether, or an isoform thereof, or a mixture thereof.

### PLANT EXTRACT

An extract containing xanthone derivatives in accordance with the invention may be prepared according to any known methods.

For example, an extract according to the invention may be prepared according to the following procedures.

A part of a plant, for example the stem or leaf or bark of a plant liable to contain a xanthone derivative in accordance with the invention, such as *Allanblackia sp*., is dried, cut, crushed and extracted by maceration with a mixture of a polar and non-polar solvent such as chloroform/methanol, and the like, at room temperature for 10 to 48 hours, preferably for 24 hours and then with a polar solvent, such as methanol for 2 to 6 hours, preferably for 4 hours.

The supernatant may be dry-concentrated with rotary evaporator under reduced pressure, at a temperature ranging from 20 to 100°C, preferably from 30 to 60°C.

The above-described procedure may be modified or subjected to further step to fractionate or isolate more potent fractions or compounds by conventional procedure well-known in the art, for example, the procedure disclosed in the literature (Harborne J.B. Phytochemical methods: A guide to modern techniques of plant analysis, 3rd Ed. Pp6-7, 1998).

A crude extract of the invention may further be subjected to a separating analysis method, such as chromatography, thin-layer chromatography or column chromatography.

Silica gel column chromatography with a solvent mixture mixed mobile phase with increasing polarity, for example, ranging from hexane:ethylacetate, ethylacetate to ethylacetate-methanol may be used for eluting the required fraction.

More preferably, a xanthone derivative extract according to the invention may be obtained as indicated in the examples.

According to another embodiment, a xanthone derivative of the invention may be obtained following total or hemi-synthesis by any known techniques in the art.

### PHARMACEUTICAL COMPOSITION

The term "pharmaceutical" or "medicament" refers to an agent or mixture of agents that is primarily intended to treat and/or ameliorate and/or prevent a disease or a disorder.

The term "pharmaceutically acceptable" means that which is useful in preparing a pharmaceutical composition that is generally safe, non-toxic, and neither biologically nor otherwise undesirable and includes what is acceptable for veterinary as well as human pharmaceutical use.

An "effective amount" means an amount sufficient to induce a positive modification in the condition to be regulated or treated, but low enough to avoid serious side effects. An effective amount may vary with the particular condition being treated, the age and physical condition of the end user, the severity of the condition being treated/prevented, the duration of the treatment, the nature of other treatments, the specific compound or product/composition employed, the route of administration, and like factors.

The term "subject" or "individual" (used interchangeably herein) means mammals and non-mammals. Examples of mammals include, but are not limited to: humans; non-human primates such as chimpanzees and other apes and monkey species; farm animals such as cattle, horses, sheep, goats, and swine; domestic animals such as rabbits, dogs, and cats; laboratory animals including rodents, such as rats, mice, and guinea pigs; and the like. Examples of non-mammals include, but are not limited to, birds, and the like. The term "subject" or "individual" does not denote a particular age or sex.

A xanthone derivative of the present invention may be administered in an effective amount by any of the accepted modes of administration in the art.

In one embodiment, a xanthone derivative may be used in a medicament intended to be administered by oral, nasal, sublingual, aural, ophthalmic, topical, rectal, vaginal, urethral, or parenteral injection route.

Suitable concentration may range from 0,0001 mg/kg/d to 50 mg/kg/d, in particular from 0,001 mg/kg/d to 5 mg/kg/d and more particularly from 0,01 to 0,5 mg/kg/d, depending upon numerous factors such as the age and relative health of the subject, the potency of the formulation used, and the indication towards which the administration is directed. One of ordinary skill in the art of therapeutic formulations will be able, without undue experimentation and in reliance upon personal knowledge and the disclosure of this Application, to ascertain a therapeutically effective amount of a xanthone derivative of the invention for a given indication.

A medicament of the invention may be intended to be administered separately, sequentially or simultaneously with a chemotherapeutic agent or a radiotherapeutic regimen.

A medicament of the invention may be formulated with any known suitable pharmaceutically acceptable carrier according to the dose, the galenic form, the route of administration and the likes.

As used herein, "pharmaceutically acceptable carrier" includes any and all solvents, dispersion media, coatings, antibacterial and antifungal agents, isotonic and absorption delaying agents and the like. Except insofar as any conventional media or agent is incompatible with the active ingredient, its use in a medicament of the invention is contemplated.

A pharmaceutically acceptable carrier may be chosen according to the dose, the galenic form, the route of administration and the likes.

A medicament of the invention may be in the form of tablets, pills, powders, lozenges, sachets, cachets, elixirs, suspensions, emulsions, solutions, syrups, aerosols, sprays, ointments, gels, creams, sticks, lotions, pastes, soft and hard gelatin capsules, suppositories, sterile injectable solutions, sterile packaged powders and the likes.

### KIT-OF-PARTS

The invention is also directed to a novel kit-of-parts that is suitable for use in the treatment of cancers.

A kit of the invention may comprise (i) a xanthone derivative, as defined above, and (ii) a chemotherapeutic agent, each of (i) and (ii) being laid out to be administered separately or sequentially or simultaneously.

As example of chemotherapeutic agents that may be suitable for the invention, one may mention chemotherapeutic agents chosen from alkylating agents, anti-metabolite agents, anti-tumor antibiotics, plant alkaloids, steroid hormones, monoclonal antibodies, and mixtures thereof.

As example of alkylating agents that may be used in accordance with the invention, one may mention chlorambucil and cyclophosphamide.

As example of anti-metabolite agents that may be used in accordance with the invention, one may mention fludarabine, 6-mercaptopurine and 5-fluorouracil (5 FU).

As example of anti-tumor antibiotics that may be used in accordance with the invention, one may mention the mitomycin-C, the bleomycin, and the anthracyclines such as the doxorubicine.

As example of plant alkaloids that may be used in accordance with the invention, one may mention vincristine and vinblastine.

As example of steroid hormones that may be used in accordance with the invention, one may mention tamoxiphen.

As example of monoclonal antibodies that may be used in accordance with the invention, one may mention rituximab and alemtuzumab.

### METHODS OF TREATMENT

According to one embodiment, the instant invention relates to a method for preventing and/or treating a cancer chosen from chronic leukemias comprising at least the step of administering to an individual in need thereof at least an effective amount of at least one xanthone derivative in accordance with the invention.

The cancer may be as previously described.

According to one embodiment, a xanthone derivative in accordance with the invention may be administered separately, sequentially or simultaneously with a chemotherapeutic agent or a radiotherapeutic regimen.

A chemotherapeutic agent may be as above-described.

A radiotherapeutic regimen may be administered by exposing an individual in need thereof to a source of ionizing radiation such as X-ray, gamma-ray or beta-ray.

A source of ionizing radiation that may convene to the invention may be, for example external source such as radioactive cobalt or a digital linear accelerator producing X-rays or an administrated source under the form of an isotope such as for example from ¹⁴C, ³H, or ¹²⁵I, ¹³¹I, ³²P, ⁸⁹Sr, ⁹⁰Y_{.}

For example, the isotopes may be administered as radio-labeled antibodies.

The present invention will be better understood by referring to the following examples which are provided for illustrative purpose only and should not be interpreted as limiting in any manner the instant invention.

### FIGURES

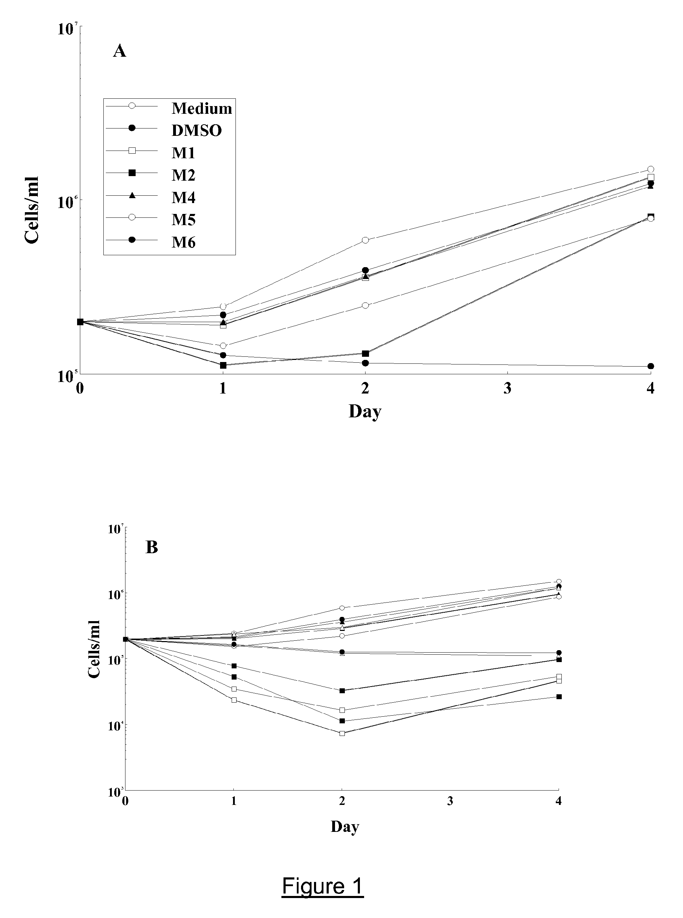

**Figure 1****:** The antiproliferative effects of M1, M2, M4, M5 and M6 were compared as indicated in the Examples Section on ESKOL cells. ESKOL cells were seeded at 2 x 10⁵ cells/ml in the wells of micro-titration plate in the presence or absence (medium alone: ○, DMSO: ●) of M1 (□), M2 (■), M4 (▲), M5 (●), M6 (○) at the concentrations of 1 µg/ml (A) and 10 µg/ml (B). After various times of incubation (1, 2 or 4 days), aliquots were harvested and the concentration of viable cells was measured in a Coulter counter as indicated in the examples.

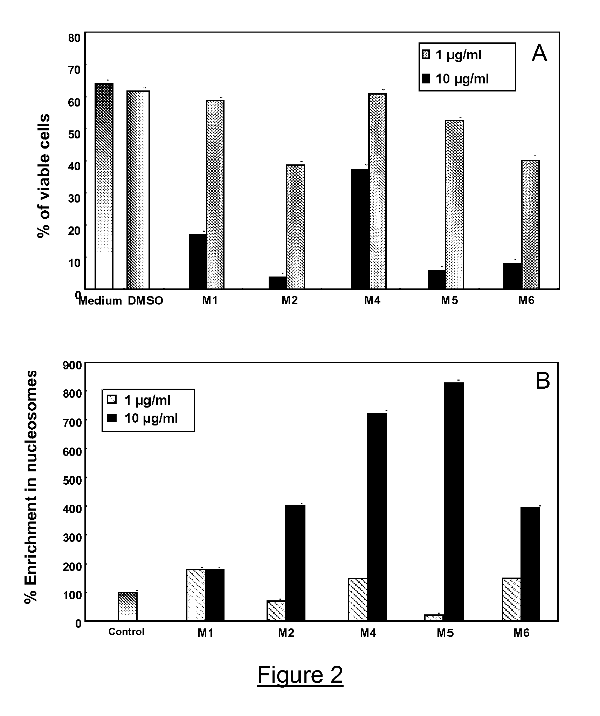

**Figure 2****:** The effects of the molecules M1, M2, M4, M5 and M6 on the viability and apoptosis induction of ESKOL cells were evaluated as indicated in the Examples Section.
   A/ ESKOL cells were incubated in the absence (gray bars) or in the presence of 1µg/ml (shaded bars) or 10µg/ml (black bars) of the various molecules. After 2 days of incubation, the viability of the recovered cells was measured with a Coulter counter by estimating the ratio of viable/total cells as indicated in the Examples Section.
   B/ The percentage of enrichment in cytoplasmic nucleosomes was evaluated simultaneously as a surrogate for DNA cleavage.

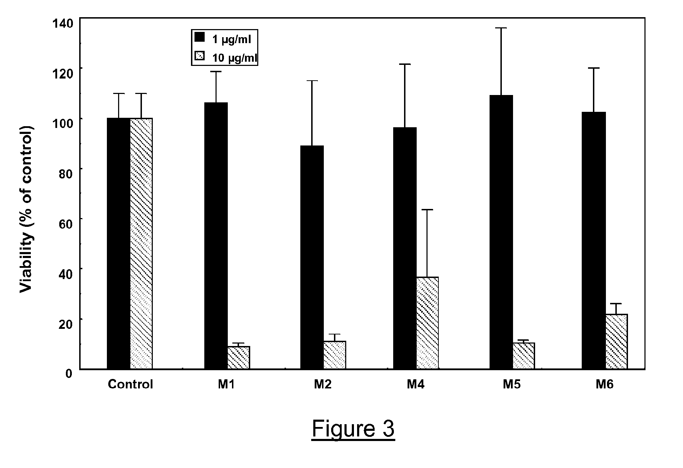

**Figure 3****:** The effect of the molecules M1, M2, M4, M5 and M6 on the viability of B-CLL cells was determined. Tumour cells freshly isolated from B-CLL patients were suspended at 2 x 10⁶/ml in complete RPMI-1640 medium and incubated for 48 hours in the presence or absence of either 1 µg/ml (black bars) or 10 µg/ml (gray bars) of the various molecules. MTT was incorporated into the cells during the last 5 hours of incubation and the viability was measured by recording the formation of the formazan precipitate as described in the Examples section. The results are expressed as the percentage of the viability of the treated cells in comparison with control cells incubated with medium alone taken as 100% and represent the mean ± SE of 6 B-CLL patients.

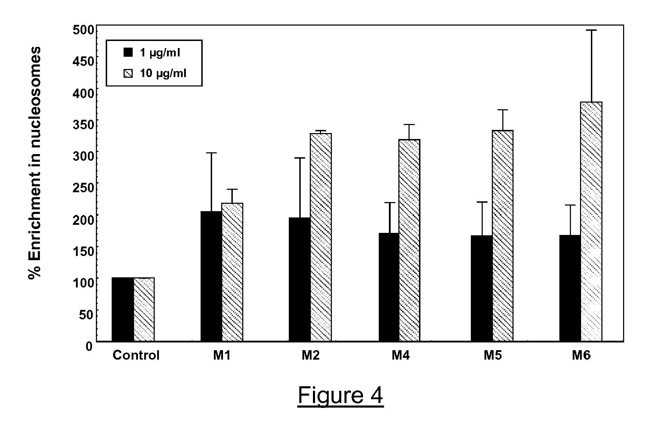

**Figure 4****:** The effect of the molecules M1, M2, M4, M5 and M6 on the enrichment in cytoplasmic nucleosomes in B-CLL cells was determined as indicated in the Examples. Leukaemia cells from 6 B-CLL patients were incubated for 24 hours in the presence of culture medium alone (control) or containing 1 µg/ml (black bars) or 10 µg/ml (grey bars) of the various molecules. DNA fragmentation was evaluated by the release of cytoplasmic nucleosomes as indicated in the Examples section. Results are expressed as the percentages (mean ± SE) of nucleosome enrichment in the cytoplasm, as compared to control cells taken as 100%.

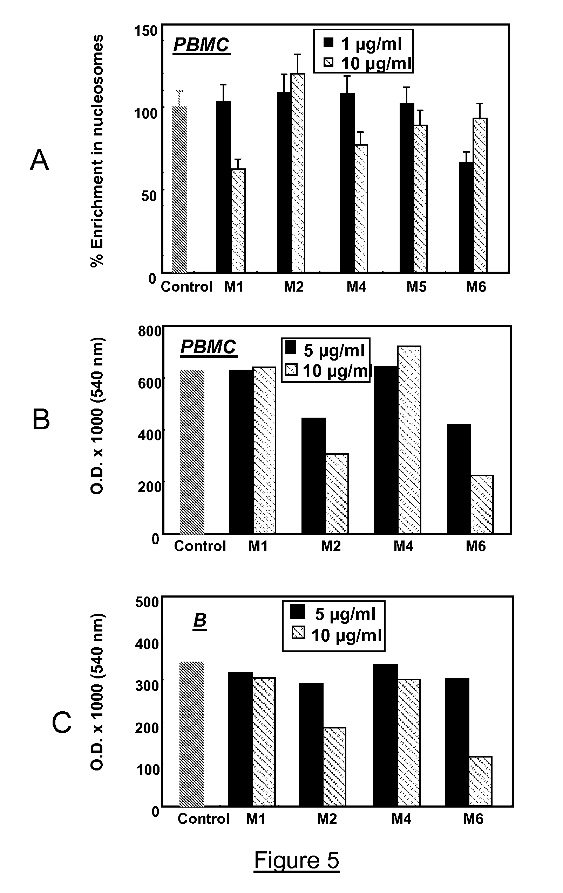

**Figure 5****:** The effect of the compounds M1, M2, M4, M5 and M6 on the viability and apoptosis of normal lymphocytes was evaluated as indicated in the Examples section. A/ PBMC isolated from the blood of normal volunteers were incubated at 2 x 10⁶/ml for 48 hours in the presence of medium alone (grey bar) or containing 1 µg/ml (black bar) or 10 µg/ml (slashed bars) of the different compounds. At the end of the incubation time, the percentage of enrichment in cytoplasmic nucleosomes was evaluated as indicated in the Examples Section. PBMC (A) and purified B lymphocytes (C) from normal blood donors were incubated for 48 hours with medium alone (grey bar ) or containing 5 µg/ml (black bar)or 10 µg/ml (slashed bar) of the selected reagents M1, M2, M4 and M6. Viability was estimated after labelling the cells with a 5 hours pulse of MTT and determination of the absorbance at 540 nm as indicated in the Examples Section.

### EXAMPLES

### REAGENTS

For the following experiments, 1 mg/ml stock solutions of the M1, M2, M4, M5 and M6 xanthone derivatives were made in DMSO. Flavopiridol, used as a positive control of apoptosis induction, was provided by Aventis Pharmaceuticals (Bridgewater, NJ, USA). A 10mM stock solution was prepared in DMSO, aliquoted and kept at -20°C. In some experiments, combretastatin analogues, either pro-apoptotic (J1 and J3) or not (J2) for B-CLL cells were also included as controls. The broadly specific caspase inhibitor z-VAD-fmk was obtained from Biomol (Plymouth Meeting, PA, USA). Except when otherwise stated, FITC-conjugated antibodies were purchased from Becton Dickinson (Mountain View, CA, USA). The other reagents and chemicals were from Sigma (St Louis, MO, USA).

Statistical analysis was performed using the Statview software; the unpaired two tail t test was used for the comparison of test and control groups.

### EXAMPLE 1

### Isolation and purification of compounds M1, M2, M3, M4, M5 and M6

### Vegetal Materials

Three medicinal cameroonean plants of the genus *Allanblackia* belonging to the family of Guttiferae, were used for the study. The plant material used is composed of:
- Trunk bark, roots, leaves and fruits from *Allanblackia monticola*, harvested near Bagante in the West province of Cameroon.
- Roots from *Allanblackia floribunda* and *Allanblackia gabonensis,* harvested in the Mbankomo area at the summit of Mount Kala in the central province of Cameroon.

The identification of the three plants was carried out by Dr. Zapfack from the Department of Plant Physiology at the University of Yaounde I.
- Fruits from *Calophyllum inophyllum* were collected in Kribi and identified at the " Herbier national du Cameroun " (National plant collection of Cameroon) at the University of Yaounde I where a reference sample (N° 32189/SRF/Cam) has been deposited.

### Extraction

The roots of *Allanblackia floribunda* were cut into small pieces, dried and crushed. The resulting powder (5kg) was extracted by maceration, successively in the mixture of CH₂Cl₂-MeOH (1:1) for 24 hours, then in pure methanol (MeOH) for 4 hours. Each sample is dry-concentrated under reduced pressure using a rotary evaporator to afford respectively 137g for CH₂Cl₂-MeOH extract and 45 g for MeOH extract. Both extracts are pooled on the basis of analytical thin layer chromatography (TLC) to give a total extract (EAFR).

The roots of *Allanblackia gabonensis* were cut, dried and crushed. The powder obtained (1.7 kg) was extracted by maceration in MeOH at room (25°C) temperature. The evaporation of the solvent under reduced pressure gave a total extract of 98g (EAGR).

The bark of the trunks, roots, leaves and hulls of *Allanblackia monticola* were cut separately into small pieces, dried and then crushed. This has enabled us to afford respectively 3 kg of bark of the trunks, 2.5kg of leaves, 2kg of roots and 375g of hulls. The extraction by maceration in a mixture of CH₂Cl₂-MeOH (1:1) of the different powders yielded after evaporation under reduced pressure 250g of extract from trunk bark (EAM), 225g of extract from leaves (EFAM), 115g of extract from roots (ERAM) and 38 g of extract from hull (ECAM).

The pulp of the fruits from *Calophyllum inophyllum* has been cut, dried and then crushed to give 9.8kg of powder. Extraction of 5 kg by maceration with a mixture of CH₂Cl₂-MeOH (1:1) yielded after evaporation under reduced pressure 1.85 kg of an oily extract.

### Isolation and purification of M2 (α-mangostin) and M5 (norcowanin)

150g out of 250g of crude extracts from the bark of the trunks of *Allanblackia monticola* are fractionated on a " flash " column containing 200 g of silica (70-230 mesh). The elution is performed using an hexane-ethyl acetate mixture of increasing polarity. Fractions of 300 ml are collected and pooled on the basis of thin-layer chromatography (TLC) analysis. This has enabled to get 4 series indexed A, B, C and D. The B series, resulting from fractions 18-39 eluted with hexane-ethyl acetate 7.5 / 2.5 mixture, consists in a viscous mass of 25g. It is then chromatographied on a silica gel column (70-230 mesh) and eluted with a mixture of hexane-ethyl acetate of increasing polarity. Ninety five fractions of 150 ml each were collected and analysed by TLC. Based on the results of the TLC, fractions 71-80 containing 4 products are pooled. After several column chromatographies using hexane-ethyl acetate 9:1 mixture as eluent, 2 compounds were isolated in pure form. The first is constituted of 15 mg of a yellow powder soluble in acetone and is indexed AM2. The second is constituted of 300 mg of yellow crystals and is indexed AM5. Fractions 85-90 rechromatographied in a small column and eluted with hexane-ethyl acetate 8 :2 give 25 mg of yellow crystals indexed AM7.

The structures of compounds AM₅ (C₂₄H₂₆O_{6;} MW=410 g/mol; melting point = 178-180°C) and AM7 (C₂₈H₃₂O₆; MW=464 g/mol) were identified on the basis of their physical and spectral data respectively to the previously described α-mangostin (Chairungsrilerd et al, Phytochemistry, 1996a; 43 : 1099-1102) and norcowanin (Pattalung, Planta Medica 1994 ; 60 : 365-368) and are respectively referred to hereafter as M2 and M5.

### Isolation and purification of M3/Allanxanthone E.

The crude extract of the fruit of *A*. *monticola* (32g) was fractionated by column chromatography using silica gel (70-230 mesh) and eluted with n-hexane, mixtures of hexane-ethyl acetate (7.5:2.5) and (1:1), pure ethyl acetate and ethyl acetate-MeOH (7.5:2.5). A total of 75 fractions of ca 300 ml each were pooled on the basis of analytical TLC. Four main fractions were obtained A (4g), B (5g), C (8g) and D (11g), respectively. Fraction A consists of very apolar compounds (fatty alcohols). Fraction B constituted of the materials eluted with the hexane-ethyl acetate mixture (7.5:2.5) was chromatographied again on a column of silica gel. A gradient elution was conducted with hexane-ethyl acetate mixtures. A total of 50 fractions of 100 ml each were collected and pooled on the basis of analytical TLC. Pure compounds were obtained by direct crystallization or after other purifications by column chromatography. Repeated column chromatographies of fraction D were performed and eluted successively with pure CH₂Cl₂ then with the mixtures CH₂Cl₂/ MeOH (19:1) and (18:2) and have resulted in the isolation of M3 (15 mg) under as amorphous yellow crystals. This molecule was characterized by as 3',4' dehydromangostanol and named Allanxanthone E.

### Isolation and purification of compounds M6 (macluraxanthone).

150 g out of 182 g of the total extract EAFR of the roots of *Allanblackia floribunda* were subjected to a "Flash" chromatography in a column containing 500g of silica (70-230 mesh) and eluted respectively with CH₂Cl₂, a mixture of hexane-ethyl acetate (1:1), pure ethyl acetate and finally with a mixture of ethyl acetate-methanol (9:1). Twenty fractions of 400 ml each were collected and pooled on the basis of the results of analytical TLC. Fractions 50 to 53 eluted with the mixture hexane-ethyl acetate (9:1) precipitate a yellow powder, which after washing and filtration provides a pure compound indexed M6.

The structure of the compounds M6 (C₂₃H₂₂O₆ ; MW= 394 g/mol; melting point = 171°C) was established on the basis of its physical and spectral data, respectively, to the previously described Macluraxanthone (Groweiss et al, J Nat Prod. 2000; 63:1537-1539).

### Isolation and purification of compounds M1/1,3 dihydroxy-7-methoxy-2(3-methylbut-2enyl) and M2/(α-mangostin)

The crude extract of the fruit of *A*. *monticola* (32g) was fractionated by column chromatography using silica gel (70-230 mesh) and eluted with n-hexane, mixtures of hexane-ethyl acetate (7.5:2.5) and (1:1), pure ethyl acetate and ethyl acetate-MeOH (7.5:2.5). A total of 75 fractions of ca 300 ml each were pooled on the basis of analytical TLC. Four main fractions were obtained A (4g), B (5g), C (8g) and D (11g), respectively. Fraction A consists of very apolar compounds (fatty alcohols). Fraction B constituted of the materials eluted with the hexane-ethyl acetate mixture (7.5:2.5) was chromatographied again on a column of silica gel. A gradient elution was conducted with hexane-ethyl acetate mixtures. A total of 50 fractions of 100 ml each were collected and pooled on the basis of analytical TLC. Pure compounds were obtained by direct crystallization or after other purifications by column chromatography. Fractions (18-23) eluted with hexane-ethyl acetate (18:2) were subjected to preparative thin layer chromatography and provided the compound M1 that was identified to 1,3 dihydroxy-7-methoxy-2(3-methylbut-2enyl) xanthone (Sen et al, Phytochemistry 1981, 20, 183-185). Fraction C, obtained from the fractions eluted with hexane-ethyl acetate (1:1) were treated the same way as Fraction B and resulted in the isolation of 400 mg of yellow crystals of a compound that was identified as M2/α-mangostin already described above.

### Isolation and purification of compound M4/(allanxanthone C)

The crude extract of the leaves of *A*. *Monticola* (200 g) was fractionated by chromatography column using silica gel (70-230 mesh) and eluted with n-hexane and a mixture of hexane-ethyl acetate (7.5:2.5), (1:1), pure ethyl acetate and a mixture of ethyl acetate-MeOH (7.5:2.5). A total of 102 fractions of ca 400 ml each were pooled on the basis of analytical TLC, leading to four main fractions indexed A (20g), B (26g), C (38g) and D (91g). Repeated column chromatographies of fraction C were performed and eluted successively with pure CH₂Cl₂, then with a mixture of CH₂Cl₂-MeOH (19:1) and have resulted in the obtention of 75 mg of a greenish oil indexed M4 or allanxanthone C (C₂₈H₃₂O; MW = 464) (Azebaze et al., Chem Pharm Bull. 2006; 54: 111-113).

### EXAMPLE II

### Antiproliferative and pro-apoptotic effects of Allanblackia derivatives on ESKOL cell line.

The anti-proliferative effects of the isolated molecules were investigated on the ESKOL cell line derived from a hairy cell leukemia patient, a chronic B cell malignancy (Harvey et al, Leuk Res. 1991; 15: 733-744).

Cells were routinely cultured at seeding densities of 2x10⁶ cells/ml in RPMI-1640 medium supplemented with 2mM glutamine, 1mM sodium pyruvate, 100 IU/ml penicillin, 100 µg/ml streptomycin and 10% FCS (PAA Laboratories, Pasching, Austria) and cultures were performed at 37°C in an humidified atmosphere containing 5% CO₂.

For the assay, ESKOL cells were seeded at 2x10⁵/ml in 24-well microtitration plate, together with two concentrations (1 and 10 µg/ml) of the three xanthones or 1% DMSO (solvent) as control. After 1, 2 and 4 days of culture, aliquots of cells were collected and the cell concentration was evaluated by counting the number of viable cells with a Coulter Multisizer counter Z2 (Coultronics, Margency, France) allowing the discrimination between viable and dead cells and debris according to size distribution.

Cells were cultured in duplicates or triplicates and each experiment repeated 2 to 5 times.

As seen in Figure 1A and 1B, M1, M2, M4, M5 and M6 markedly and dose-dependently slowed down the proliferation of these cells.

The impairment in cell multiplication was accompanied by a reduction in the percentage of viable cells, as shown in Figure 2A after 48 hours of incubation in the presence of the various reagents. The latter could be attributed for the most part to apoptosis induction, as attested by the enrichment in cytoplasmic nucleosomes, an internucleosomal cleavage of DNA being a hallmark of cell death by apoptosis (Figure 2B). M2, M4, M5 and M6 were found to be potent inducers of DNA breakdown.

The induction of apoptosis was further confirmed by assessing the percentage of cells expressing phosphatidylserine at the outer leaflet of the plasma membrane by flow cytometry.

The phosphatidylserine (PS) externalisation, a membrane marker of cells undergoing apoptosis, was quantified by specific binding of FITC-conjugated annexin V (Bender Medsystems, Vienna, Austria), with or without simultaneous labelling with propidium iodide (PI), according to a modification of the technique described by Koopman (Koopman et al, Blood 1994; 84:1415-1420). The percentages of annexin V-FITC positive and PI negative cells were determined by cytometry on an EPICS Altra flow cytometer (Beckman Coulter).

The ESKOL cells (10⁶/ml) were cultured in the absence (C = control) or the presence of 1 µg/ml of the various *Allanblackia* molecules. After 24 hours of incubation, the percentage of cells labelled with Annexin V-FITC was measured by flow cytometry as previously indicated. Results are expressed after subtraction of the value for control unstimulated cells. P values were estimated by Kolmogorov-Smirnov analysis using the Statview software.

As presented in Table 1, M2 and M6, and to a lesser degree M4 and M5 elicited a significant increase in the percentage of Annexin V positive cells.

**Table 1. Effect of the various derivatives on the percentage of ESKOL cells labelled with Annexin V**

| Reagent | M1 | M2 | M4 | M5 | M6 |
|---|---|---|---|---|---|
| % AnnexinV⁺ cells | 5.6 | 15.0 | 10.2 | 9.4 | 36.5 |
| p | NS | ** | * | * | *** |

| | | | | | |
|---|---|---|---|---|---|
| NS: not significant * p <0.05 ** p < 0.02 *** p < 0.01 | | | | | |

### EXAMPLE III

### Allanblackia-derived molecules promote a loss of viability and induce apoptosis in cells from B-CLL patients.

Blood samples from B-CLL patients were obtained from the Haematology Department of Hôtel-Dieu hospital (Paris, France) after written informed consent, in accordance with the rules and tenets of the revised Helsinki protocol. Diagnosis was established according to standard clinical and international CLL workshop criteria, including lymphocyte morphology and co-expression of CD5, CD20 and CD23 antigens. A total of 12 patients (6 men and 6 women) with an age ranging 50-84 years (mean ± SD: 68 ± 8.8 years) were selected and the time since diagnosis varied between 0 (newly diagnosed patients) and 10 years. The patients were randomly chosen for each type of experiment inasmuch as CD38 and ZAP-70 expression, cytogenetic analysis and mutational V_{H} status were only available for a fraction of them, thus hampering a risk-group analysis. The leukemia B-cells were isolated with purity greater than 96%, as previously described (Zhao et al, Blood 1998; 92: 1031-1043). All the experiments were performed with freshly purified B-CLL leukemia cells.

Blood-derived B-CLL cells do not proliferate, inasmuch as 95-98% of them are arrested at the G₀/G₁ stage of the cell cycle.

M1, M2, M4, M5 and M6 were thus tested for their possible action on the viability of these leukemia cells by the MTT assay that relies on the integrity of the mitochondrial succinyl dehydrogenase.

Leukemia cells from B-CLL patients were adjusted at 2x10⁶/ml and 200 µl aliquots were distributed in triplicate samples in the wells of a 96-well microtitration plate and cultured in the absence or presence of the various reagents. After 18 hours of incubation at 37°C in a humidified atmosphere containing 5%CO₂, 20 µl of a 5 mg/ml solution of MTT (Sigma, St Louis, Mo, USA) were added to the wells for an additional 6 hours incubation. The microplates were centrifuged at 400 x g for 20 min, the supernatants carefully discarded and 200 µl of DMSO were added to the wells to dissolve the precipitates of formazan. The optical density was measured with the use of a microplate reader (Victor-2, Wallac, Perkin Elmer, Norwalk, MT, USA) at the wavelength of 540 nm.

As seen in Figure 3, no significant effect was observed at the concentration of 1 µg/ml, but a marked reduction in the viability of B-CLL cells was observed at the concentration of 10 µg/ml for the compounds M1, M2, M4, M5 and M6.

In order to assess whether this reduction in viability resulted from apoptosis induction, DNA fragmentation was measured in the treated cells by estimating the release of cytoplasmic nucleosomes.

The detection of cytoplasmic histone-associated DNA fragments (mono- and oligonucleosomes) was performed in cell lysates from aliquots of 20,000 cells using an ELISA with anti-histone and anti-DNA fragment mAbs (Cell Death Detection ELISA^{PLUS}, Roche Diagnostics, Indianapolis, IN, USA) as previously described (Billard et al, Leuk Lymphoma 2002; 43: 1991-2002).

Indeed, incubation of B-CLL cells with the various reagents was found to elicit an increase in nucleosomes fragmentation when compared with untreated cells, as seen in Figure 4. At the concentration of 10 µg/ml, the most potent compounds (M2, M4, M5, M6) stimulated more than 3 fold the release of nucleosomes in the cytoplasm of the treated cells after 24 hours of incubation.

Induction of apoptosis was also confirmed by measuring the percentage of cells labelled with AnnexinV-FITC as a marker of phosphatidylserine externalisation. Labeling of cells with Annexin V-FTC was performed as above-indicated.

Despite the well-known high level of spontaneous apoptosis that occurs *ex vivo* in B-CLL cells, treatment with the various molecules resulted for M2, M4, M5, M6 in a significant increase (10-30%) in the percentage of AnnexinV⁺/PI- cells.

### EXAMPLE IV

### Effect of compounds M1, M2, M4, M5 and M6 on normal PBMC and B lymphocytes

The compounds M1, M2, M4, M5 and M6 were then tested for their potential pro-apoptotic effects on peripheral blood mononuclear cells obtained from normal blood donors.

Blood samples from healthy donors were obtained from the Institut Français du Sang as residues from platelet preparations. The healthy blood donors were under 60 years old, according to the French legislation, and therefore they could not be exactly age-matched with the B-CLL patients. PBMC were prepared as described elsewhere (Zhao et al, Blood 1998; 92: 1031-1043) and normal B-lymphocytes were purified by positive selection on anti-CD19-coated magnetic beads according to the specifications of the manufacturer (Dynal, Oslo, Norway) (Kern et al, Blood 2004; 103: 679-688). Their purity usually ranged from 92% to 95%, as estimated by labelling with an anti-CD20-phycoerythrin (PE) antibody, and monocyte contamination never exceeded 2% (Kern et al, Blood 2004; 103: 679-688).

The DNA fragmentation assay was performed as above-indicated.

As seen in Figure 5A, no significant increase in the percentage of cytoplasmic nucleosome enrichment was observed with the various molecules tested up to 10 µg/ml.

The most active derivatives on CLL were also screened by the MTT test as above-indicated for their cytotoxic effect, both on normal PBMC (Figure 5B) and purified B-lymphocytes (Figure 5C). These cells were found somewhat sensitive to M2 and M6 in a dose-dependent way, whereas no toxic effect was observed with M1 and M4 up to 10µg/ml.

## Claims

1. A xanthone derivative of general formula (IA) or (IB): 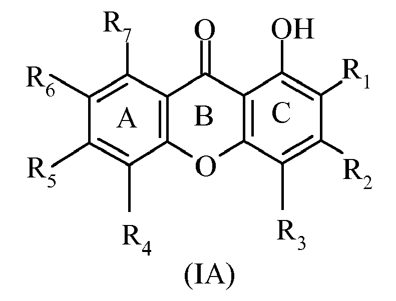 or 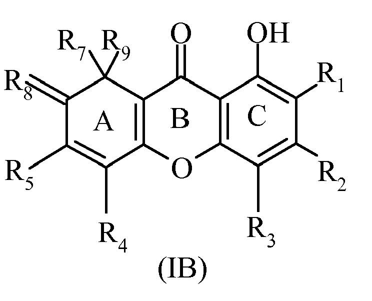 wherein
- R₁, R₃, R₇ and R₉ are, independently of each other, H or a linear, branched or cyclic, saturated or unsaturated C₁-C₁₂ alkyl group, with the proviso that at least one of R₁, R₃ or R₇ is an alkyl group as above-defined,
- R₂, R₄ and R₅ are, independently of each other, H, -OH, -NH₂ or -SH, or
- R₁ and R₂ form together a 5 to 7-membered ring fused with ring C, said ring being saturated or unsaturated, and optionally comprising at least one heteroatom chosen from O, N or S, and optionally being substituted with one or more linear, branched or cyclic, saturated or unsaturated C₁-C₆ alkyl groups, R₃ R₄, R₅, R₇ and R₉ being as above-defined,
- R₆ is a group chosen among linear or branched, saturated or unsaturated C₁-C₄ alcoxy, C₁-C₄ alkyl- or dialkyl-amino, or C₁-C₄ alkyl-imido groups,
- R₈ is chosen among an oxygen, an imine or a thioether,
or a pharmaceutically acceptable salt, an ester, an ether, or an isoform thereof, or a mixture thereof,
for use as a medicament for the prevention and/or the treatment of a cancer chosen from chronic leukemias.

2. The xanthone derivative according to the preceding claim, wherein one or more of R₁, R₃, R₇ or R₉ is a linear, branched or cyclic, saturated or unsaturated C₂-C₁₂, C₃-C₁₀ or C₅-C₁₀ alkyl group, and in particular is a 3-methyl-but-2-enyl group or a 3,7-diméhtyl-oct-2,6-dienyl group.

3. The xanthone derivative according to claim 1, wherein R₁ and R₂ form together a 6 membered ring fused to ring C, said 6 membered ring optionally comprising at least one oxygen and/or at least one unsaturation and/or being optionally substituted with one or more linear or branched, saturated or unsaturated C₁-C₃ alkyl groups.

4. The xanthone derivative according to claim 1 or 3, wherein said ring is substituted with one or more methyl groups.

5. The xanthone derivative according to anyone of the preceding claims, wherein at least one of R₂, R₄ or R₅ is OH.

6. The xanthone derivative according to anyone of the preceding claims, wherein said xanthone derivative comprise at least 2 hydroxyl groups, and in particular at least 3 hydroxyl groups, and more particularly at least 4 hydroxyl groups.

7. The xanthone derivative according to anyone of the preceding claims, wherein R₆ is a C₁-C₂ alcoxy group, and in particular a methoxy group.

8. The xanthone derivative according to anyone of the preceding claims, wherein R₈ is an oxygen.

9. The xanthone derivative according to anyone of the preceding claims, wherein said xanthone derivative is chosen from a compound of formula M1/(II), M2/(III), M4/(IV), M5/(V) and M6/(VI): 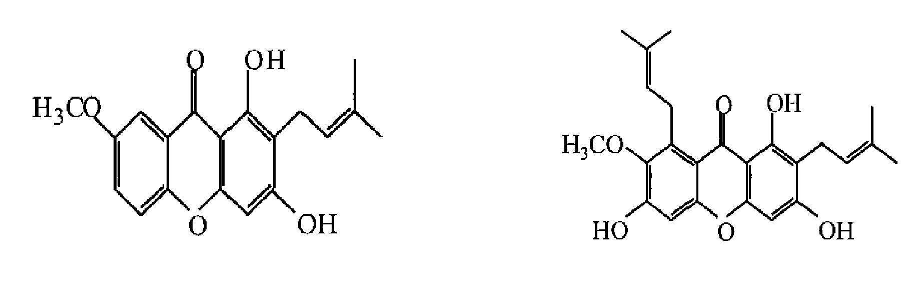 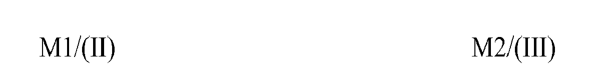 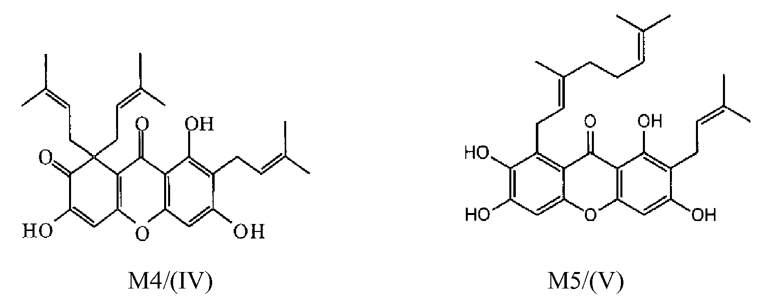 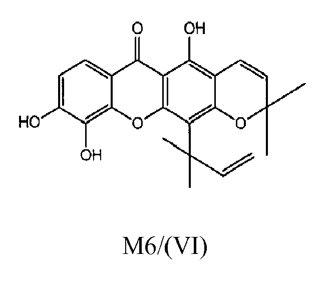 or a pharmaceutically acceptable salt, an ester, an ether, or an isoform thereof, or a mixture thereof.

10. The xanthone derivative according to anyone of claims the preceding claims, wherein said cancer is a chronic leukemia chosen from B-cells chronic lymphoid leukemia (B-CLL) or B-lymphoma

11. The xanthone derivative according to anyone of claims the preceding claims, wherein said cancer is resistant to chemotherapy or radiotherapy.

12. The xanthone derivative according to anyone of the preceding claims, wherein said medicament is intended to sensitize cancer cells to a chemotherapeutic agent or a radiotherapeutic regimen.

13. A kit-of-parts comprising (i) a xanthone derivative as defined according to anyone of claims 1 to 9, and (ii) a chemotherapeutic agent, each of (i) and (ii) being laid out in a separate dosage form unit.

14. The kit-of-parts according to the preceding claim, wherein said (i) xanthone derivative and said (ii) chemotherapeutic agent are laid out to be administered separately, sequentially or simultaneously.

15. A method for preventing and/or treating a cancer chosen from chronic leukemias comprising at least the step of administering to an individual in need thereof at least an effective amount of at least one xanthone derivative as defined according to anyone of claims 1 to 9.
